(19) Europäisches Patentamt / European Patent Office / Office européen des brevets

(11) EP 1 779 836 B1

(12) **EUROPÄISCHE PATENTSCHRIFT**

(45) Veröffentlichungstag und Bekanntmachung des Hinweises auf die Patenterteilung:
**11.12.2013 Patentblatt 2013/50**

(51) Int Cl.:
***A61K 8/06*** *(2006.01)* ***A61K 8/73*** *(2006.01)*
***A61K 8/81*** *(2006.01)* ***A61Q 19/00*** *(2006.01)*
***A61Q 17/04*** *(2006.01)*

(21) Anmeldenummer: **06020997.0**

(22) Anmeldetag: **06.10.2006**

(54) **Hydrodispersion mit einem Gehalt an Hydroxypropylguar und Acrylatpolymeren**

Hydrodispersion comprising a hydroxypropylguar and a acrylate polymer

Hydrodispersion comprenant un hydroxypropylguar et un polyacrylate

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **27.10.2005 DE 102005051859**

(43) Veröffentlichungstag der Anmeldung:
**02.05.2007 Patentblatt 2007/18**

(73) Patentinhaber: **Beiersdorf Aktiengesellschaft**
**20253 Hamburg (DE)**

(72) Erfinder:
- **von Thaden, Stephanie**
**20255 Hamburg (DE)**
- **Schäfer, Andreas, Dr.**
**22299 Hamburg (DE)**
- **Bauer, Anja**
**22459 Hamburg (DE)**
- **Boenisch, Alexandra**
**20257 Hamburg (DE)**
- **Viala, Sophie, Dr.**
**20257 Hamburg (DE)**

(56) Entgegenhaltungen:
**EP-A- 1 291 007**
**WO-A-94/18935**
**WO-A-99/22701**
**DE-A1- 19 727 508**
**FR-A1- 2 779 638**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 779 836 B1

## Beschreibung

Die vorliegende Erfindung betrifft Hydrodispersionen mit einem Gehalt an Hydroxypropylguar und Acrylatpolymeren.

Die vorliegende Erfindung betrifft in einer besonderen Ausführungsform kosmetische oder pharmazeutische Zubereitungen mit vermindertem Klebrigkeitsgefühl.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht Außerdem sollen Hautpflegeprodukte vor Umwelteinflüssen, insbesondere vor Sonne und Wind, schützen und die Hautalterung verzögern.

Medizinische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

Weiterhin war es eine Aufgabe der Erfindung, kosmetische Grundlagen für kosmetische Zubereitungen zu entwickeln, die sich durch gute Hautverträglichkeit auszeichnen.

An sich ist die Verwendung der üblichen kosmetischen Emulgatoren unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mallorca-Akne"genannt. Eine Aufgabe der vorliegenden Erfindung war daher, emulgatorfreie Sonnenschutzprodukte zu entwickeln, welche sich durch hohen Lichtschutzfaktors bzw. UV-A-Schutzleistung auszeichnen.

Emulgatorfreie Lichtschutzpräparate auf Basis sogenannter Hydrodispersionen sind seit einiger Zeit für den Verbraucher zugängig. Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen metastabile Systeme dar, und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Obwohl dieser Formulierungstyp gegenüber den herkömmlichen Lichtschutzformulierungen den Vorteil der Emulgatorfreiheit bietet, gibt es andererseits auch einige Punkte, welche der Verbesserung bedürfen. So ist für eine gute Lichtschutzwirkung solcher Präparate eine vergleichsweise hohe Konzentration an UV-Filtern notwendig. Darüberhinaus fühlen sich solche Präparate im Vergleich zu Emulsionen und Lichtschutzölen klebrig an.

Ein weiterer Nachteil von Hydrodispersionen, insbesondere solcher, die Acrylate als Gelbildner enthalten, ist der unvorteilhafte Abrieb, insbesondere nach dem Baden, wo die auf die Haut aufgetragene Hydrogelschicht leicht und in unschöner Weise ("Röllchenbildung") abgetragen wird.

Ein Nachteil insbesondere von Öl-in-Wasser-Systemen ist oft deren mangelnde Stabilität gegenüber höheren Elektrolytkonzentrationen, was sich in Phasentrennung äußert. Dies kann zwar auch bei W/O-EMulsionen gelegentlich zu Problemen führen, tritt dort aber bei weitem nicht so in den Vordergrund wie bei O/W-Systemen, insbesondere bei emulgatorfreien Hydrodispersionen.

Es ist andererseits oft wünschenswert, bestimmte Elektrolyte einzusetzen, um deren sonstige physikalische, chemische bzw. physiologische Eigenschaften nutzen zu können.

Eine weitere Aufgabe der vorliegenden Erfindung war es also, Lösungswege zu kosmetischen oder dermatologischen Emulsionen, insbesondere O/W-Emulsionen, aufzudecken, welche gegenüber erhöhten Elektrolytkonzentrationen - bzw. erhöhten Ionenstärken - stabil sind.

Bekannt ist an sich, daß der Zusatz von Hydroxypropylguar die Elektrolytstabilität von Emulsionen verbessert.

Hydroxypropylguar ist der Sammelbegriff für die Reaktionsprodukte, die bei der alkalisch katalysierten Veretherung von Guarmehl mit Propylenoxid entstehen. Dabei werden die Hydroxywasserstoffatome des Guarans teilweise durch Gruppen des Typs

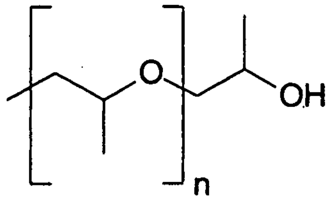

mit $n \geq 0$ ersetzt.

Es war indessen überraschend und für den Fachmann nicht vorauszusehen, daß kosmetische und dermatologische Zubereitungen in Form von Hydrodispersionen von Öltröpfchen in einer Wasserphase, welche höchstens 1 Gew.-%, bezogen auf das Gesamtgewicht der Zubereitungen, an Emulgatoren enthalten, bevorzugt aber weitgehend frei von Emulgatoren sind, wobei die wäßrige Phase aufweist:

(a) einen Gehalt an Hydroxypropylguar sowie
(b) einen Gehalt an einem oder mehreren spezifischen Acrylatpolymeren die Lösung dieser Aufgaben darstellen.

Im Rahmen der vorliegenden Offenbarung werden die Zubereitungen gemäß der Erfindung auch schlicht "Hydrodispersionen" genannt.

Die Hydrodispersionen gemäß der Erfindung zeichnen sich durch hohe Stabilität gegenüber der Gegenwart von Elektrolyten aus, selbst in völliger Abwesenheit von Emulgatoren oder sonstigen stabilisierend wirkenden Agentien.

Überraschend und nicht vorhersehbar war, daß bei Befolgung der hiermit offenbarten Lehre zum technischen Handeln in jeglicher Hinsicht überaus befriedigende Präparate erhältlich sind.

Ferner war überraschend, daß die Klebrigkeit der Hydrodispersionen bei Befolgung der hiermit offenbarten Lehre zum technischen Handeln drastisch vermindert und das unvorteilhafte Abriebverhalten stark verbessert werden kann.

Die Konzentration an Hydroxypropylguar in den Zubereitungen gemäß der Erfindung beträgt vorzugsweise 0,001 bis 5 Gew.-%, besonders bevorzugt 0,05 - 2,5 Gew.-%, insbesondere 0,1 - 1 Gew.-%, bezogen auf das Gesamtgewicht der Zubereitung.

Die erfindungsgemäßen Polyacrylate sind Acrylat-Alkylacrylat-Copolymere, die sich durch die folgende Struktur auszeichnen:

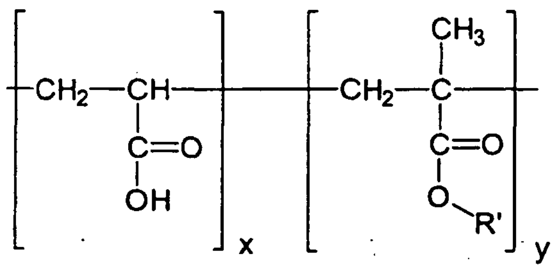

Darin stellen R' einen $C_{10-30}$ Alkylrest und x und y Zahlen dar, welche den jeweiligen stöchiometrischen Anteil der jeweiligen Comonomere symbolisieren. Es sind Copolymere aus $C_{10-30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit, und sie tragen die INCI-Bezeichnung "Acrylates/$C_{10-30}$ Alkyl Acrylate Crosspolymer". Insbesondere vorteilhaft sind die unter den Handelsbezeichnungen Pemulen TR1 und Pemulen TR2 bei der B. F. Goodrich Company erhältlichen.

Die Konzentration an einem oder mehreren Polyacrylaten, bevorzugt an Verbindungen, die die INCI-Bezeichnung "Acrylates/C $_{10-30}$ Alkyl Acrylate Crosspolymer" tragen, in den Zubereitungen gemäß der Erfindung beträgt vorzugsweise 0,001 bis 10 Gew.-%, besonders bevorzugt 0,05 - 5 Gew.-%, insbesondere 0,1 - 2,5 Gew.-%, bezogen auf

das Gesamtgewicht der Zubereitung.

Es ist von besonderem Vorteil das Gewichtsverhältnis von Hydroxypropylguar zu einem oder mehreren Polyacrylaten in den Zubereitungen gemäß der Erfindung aus dem Bereich von 100 : 1 bis 1 : 100, bevorzugt 10 : 1 bis 1 : 50, insbesondere bevorzugt aus dem Bereich von 1 : 1 bis 1 : 20 zu wählen.

Vorteilhaft zeichnen sich die Hydrodispersionen gemäß der Erfindung durch einen zusätzlichen Gehalt an einem oder mehreren Elektrolyten aus.

Erfindungsgemäß werden der oder die Elektrolyte vorteilhaft gewählt aus der Gruppe der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren und deren Salze und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat Vitamin C, Kreatin, Taurin, 8-Hexadecan-1,16-dicarbonsäure (Dioic Acid), Aluminiumchlorohydrate, Folsäure und Mischungen daraus.

Erfindungsgemäß werden der oder die Elektrolyte ferner vorteilhaft gewählt aus der Gruppe der wasserlöslichen, zumeist als Alkalisalze vorliegenden wasserlöslichen UV-Filtersubstanzen, insbesondere solche, die an ihrem Molekülgerüst eine oder mehrere Sulfonsäuregruppen bzw. Sulfonatgruppen tragen. Neispiele dafür sind:

Die 2-Phenylbenzimidazol-5-sulfonsäure und ihre Salze, beispielsweise das Natrium-, Kalium-oder ihr Triethanolammonium-Salz

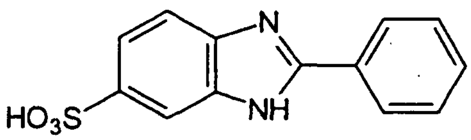

,

Sulfonsäure-Derivate von Benzophenonen, vorzugsweise 2-Hydroxy-4-methoxybenzophenon-5-sulfonsäure und ihre Salze, beispielsweise das entsprechende Natrium-, Kalium- oder Triethanolammonium-Salz:

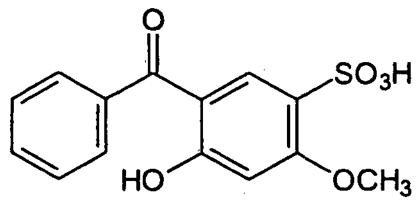

Sulfonsäure-Derivate des 3-Benzylidencamphers, wie z.B. 4-(2-Oxo-3-bornylidenmethyl)benzolsulfonsäure und ihre Salze, beispielsweise das entsprechende Natrium-, Kalium- oder Triethanolammonium-Salz:

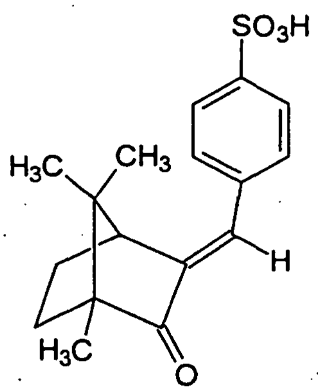

Die 2-Methyl-5-(2-oxo-3-bornylidenmethyl)benzolsulfonsäure und ihre Salze, beispielsweise das entsprechende Natrium-, Kalium- oder Triethanolammonium-Salz:

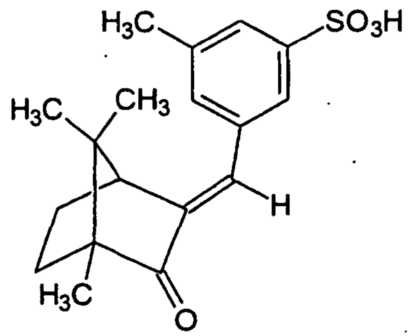

Das 1,4-di(2-oxo-10-Sulfo-3-bornylidenmethyl-)Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet:

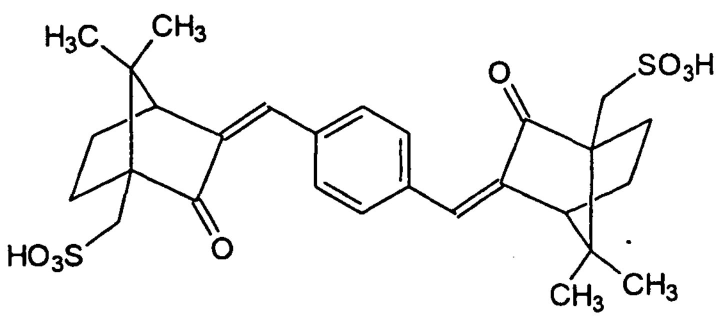

Üblicherweise werden die Konzentrationen aller Bestandteile einer kosmetischen oder dermatologischen Zubereitungen in solchen Einheiten wie Gewichts-%, Mol-% und dergleichen angegeben. Aufgrund ihrer mehr oder minder stark ausgeprägten Dissoziation in Kationen und Anionen, oftmals in mehreren Dissoziationsstufen, erscheint es manchmal zweckmäßiger für die Schilderung der vorliegenden Erfindung und ihres technischen Hintergrundes, von der Ionenstärke eines gegebenen Elektrolytes in seiner Lösung auszugehen.

Auch Phenyl-1,4-bis-(2-benzimidazyl)-3,3'-5,5'-tetrasulfonsäure und ihrer Salze, insbesondere das Phenylen-1,4-bis-(2-benzimidazyl)-3,3'-5,5'- tetrasulfonsäure, führen zu vorteilhaften Ausführungsformen der vorliegenden Erfindung.

Die Ionenstärke / einer Elektrolytlösung ist definiert als

$$I = \frac{1}{2} \sum_{i} c_i z_i^2$$

wobei $c_i$ die Konzentrationen der einzelnen Ionensorten (in mol/l) und $z_i$ deren Ladungszahlen darstellen. Die physikalische Einheit der Ionenstärke ist die einer Konzentration (mol/l).

Eine 1-%ige ( = 0,17-molare) Kochsalzlösung hat beispielsweise eine Ionenstärke I = 0,17.

Es ist erfindungsgemäß vorteilhaft, wenn die Ionenstärke der wäßrigen Phasen, in der der oder die Elektrolyte gelöst vorliegen, mindestens 0,05 mol/l, insbesondere mindestens 0,075 mol/l, besonders bevorzugt mindestens 0,10 mol/l beträgt.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 5 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Als Bestandteile der Lipidphase der erfindungsgemäßen Hydrodispersionen können vorteilhaft gewählt werden Mineralöle, Mineralwachse, Triglyceride der Caprin- oder der Caprylsäure, ferner natürliche Öle wie z.B. Rizinusöl, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, Alkylbenzoate, Silikonöle wie Dimethylpolysiloxane, Diethylpolysiloxane, Diphenylpolysiloxane sowie Mischformen daraus.

Die Ölphase der Hydrodispersionen im Sinne der vorliegenden Erfindung kann ferner vorteilhaft gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Weiterhin kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der flüssigen Fettsäuretriglyceride. Die flüssigen Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige

Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäuretriglycerid, Dicaprylylether + Butyleneglykoldicaprylat/-dicaprat, Cocoglyceride, Dicaprylylcarbonat.

Besonders vorteilhaft sind Mischungen aus $C_{12-15}$-Alkylbenzoat und 2-Ethylhexylisostearat, Mischungen aus C12-15-Alkylbenzoat und Isotridecylisononanoat sowie Mischungen aus $C_{12-15}$-Alky-Ibenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin.

Kosmetische und dermatologische Formulierungen im Sinne der Erfindung enthalten vorteilhaft einen oder mehrere übliche UV-A-Filter und/oder UV-B-Filter und/oder Breitbandfilter als Einzelsubstanzen oder in beliebigen Gemischen untereinander, in der Lipidphase und/oder in der wäßrigen Phase.

Die Gesamtmenge an UV-A-Filtersubstanzen in den fertigen kosmetischen oder dermatologischen Zubereitungen wird vorteilhaft aus dem Bereich von 0,1 bis 30 Gew.-%, bevorzugt 0,1 bis 10,0 Gew.-%, insbesondere 0,5 bis 5,0 Gew.-% gewählt, bezogen auf das Gesamtgewicht der Zubereitungen.

Die Gesamtmenge an UV-B-Filtersubstanzen in den fertigen kosmetischen oder dermatologischen Zubereitungen wird vorteilhaft aus dem Bereich von 0,1 bis 30 Gew.-%, bevorzugt 0,1 bis 20,0 Gew.-%, insbesondere 0,5 bis 15,0 Gew.-% gewählt, bezogen auf das Gesamtgewicht der Zubereitungen.

Die Gesamtmenge an Breitbandfiltersubstanzen in den fertigen kosmetischen oder dermatologischen Zubereitungen wird vorteilhaft aus dem Bereich von 0,1 bis 30 Gew.-%, bevorzugt 0,1 bis 10,0 Gew.-%, insbesondere 0,5 bis 5,0 Gew.-% gewählt, bezogen auf das Gesamtgewicht der Zubereitungen.

Die zu verwendenden UV-B-Filter können öllöslich oder wasserlöslich sein. Vorteilhafte öllösliche UV-B-Filtersubstanzen sind z.B.: 3-Benzylidencampher-Derivate, vorzugsweise 3-(4-Methylbenzyliden)campher, 3-Benzylidencampher; 4-Aminobenzoesäure-Derivate, vorzugsweise 4-(Dimethylamino)-benzoesäure(2-ethylhexyl)ester, 4-(Dimethylamino)benzoesäureamyl-ester; 2,4,6-Trianilino-(p-carbo-2'-ethyl-1'-hexyloxy)-1,3,5-triazin; Ester der Benzalmalonsäure, vorzugsweise 4-Methoxybenzalmalonsäuredi(2-ethylhexyl)ester; Ester der Zimtsäure, vorzugsweise 4-Methoxyzimtsäure(2-ethylhexyl)ester, 4-Methoxyzimtsäureisopentylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Die Liste der genannten UVB-Filter, die zusätzlich im Sinne der vorliegenden Erfindung eingesetzt werden können, soll selbstverständlich nicht limitierend sein.

Die Gesamtmenge an wasserlöslichen UV-Filtersubstanzen in den fertigen Hydrodispersionen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1** | Gew.-% |
|---|---:|
| Hydroxypropylguar | 0,3 |
| Acrylat/$C_{10-30}$ -Alkylacrylatcrosspolymer | 0,5 |
| Ethylhexyltriazon | 4,00 |
| Titandioxid | 0,50 |

(fortgesetzt)

| Beispiel 1 | Gew.-% |
|---|---|
| $C_{12-15}$ Alkyl Benzoat | 2,00 |
| Butylenglycoldicaprylat/-Dicaprat | 4,00 |
| PVP Hexadecencopolymer | 0,50 |
| Glycerin | 10,00 |
| Vitamin E Acetat | 0,50 |
| Konservierungsmittel | q.s |
| Ethanol | 3,00 |
| Parfüm, Farbstoffe | q.s. |
| Neutralisationsmittel (Natriumhydroxid, Kaliumhydroxid) | q.s |
| Kreatinin | 0,1 |
| Kreatin | 1,00 |
| Ascorbinsäure | 3,00 |
| Wasser | ad 100 |

| Beispiel 2 | Gew.-% |
|---|---|
| $C_{18-22}$ Alkylhydroxypropylguar | 0,1 |
| Acrylat/$C_{10-30}$ -Alkylacrylatcrosspolymer | 1,00 |
| Dinatriumphenyldibenzimidazoltetrasulfonat | 2,00 |
| Phenylbenzimidazolsulfonsäure | 2,00 |
| Isopropylpalmitat | 6,00 |
| Glycerin | 5,00 |
| Trinatrium EDTA | 1,00 |
| Konservierungsmittel | q.s |
| Parfüm, Farbstoffe - | q.s. |
| Neutralisationsmittel (Natriumhydroxid, Kaliumhydroxid) | q.s |
| Kreatinin | 0,1 |
| Kreatin | 0,5 |
| Wasser | ad 100 |

| Beispiel 3 | Gew.-% |
|---|---|
| Hydroxypropylguar | 0,1 |
| Acrylat/$C_{10-30}$ -Alkylacrylatcrosspolymer | 0,8 |
| Phenylbenzimidazolsulfonsäure | 2,00 |
| Ethylhexylmethoxycinnamat | 7,00 |
| Titandioxid | 1,00 |
| $C_{12-15}$ Alkyl Benzoat | 2,50 |

(fortgesetzt)

| **Beispiel 3** | Gew.-% |
|---|---|
| C18-36 Triglycerid Fettsäure | 1,00 |
| PVP Hexadecencopolymer | 0,50 |
| Glycerin - | 5,00 |
| Vitamin E Acetat | 0,50 |
| Trinatrium EDTA | 1,00 |
| Konservierungsmittel | q.s |
| Ethanol | 4,00 |
| Parfüm, Farbstoffe | q.s. |
| Neutralisationsmittel (Natriumhydroxid, Kaliumhydroxid) | q.s |
| Kreatinin | 0,1 |
| Kreatin | 0,5 |
| Octadecendicarbonsäure | 1,00 |
| Wasser | ad 100 |

| **Beispiel 4** | Gew.-% |
|---|---|
| $C_{18-22}$ Alkylhydroxypropylguar | 0,05 |
| Acrylat/$C_{10-30}$ -Alkylacrylatcrosspolymer | 0,5 |
| Dimethicon / Vinyldimethiconcrosspolymer | 5,00 |
| Bis-Ethylhexyloxyphenol-methoxyphenytriazin | 0,25 |
| Ethylhexylcyanodiphenylacrylat (Octocrylen) | 10,00 |
| Titandioxid | 2,00 |
| Octamethyltetrasiloxan (Cyclomethicone) | 7,50 |
| Ethylhexyloxyglycerin | 1,00 |
| Vitamin E Acetat | 0,25 |
| Trinatrium EDTA | 0,10 |
| Konservierungsmittel | q.s |
| Ethanol | 3,50 |
| Parfüm, Farbstoffe | q.s. |
| Neutralisationsmittel (Natriumhydroxid, Kaliumhydroxid) | q.s |
| Taurin | 1,00 |
| Magnesiumchlorid | 0,5 |
| Wasser | ad 100 |

| **Beispiel 5** | Gew.-% |
|---|---|
| Hydroxypropylguar | 0,5 |
| Acrylat/$C_{10-30}$ -Alkylacrylatcrosspolymer | 0,5 |

(fortgesetzt)

| **Beispiel 5** | Gew.-% |
|---|---|
| Dimethicon / Vinyldimethiconcrosspolymer | 3,00 |
| Dinatriumphenyldibenzimidazoltetrasulfonat | 1,00 |
| Ethylhexylmethoxycinnamat | 8,00 |
| Ethylhexylcyanodiphenylacrylat (Octocrylen) | 2,50 |
| Titandioxid | 1,00 |
| PVP Hexadecencopolymer | 1,00 |
| Ethylhexyloxyglycerin | 0,50 |
| Glycerin | 15,00 |
| Vitamin E Acetat | 1,00 |
| Konservierungsmittel | q.s |
| Ethanol | 1,00 |
| Parfüm, Farbstoffe | q.s. |
| Neutralisationsmittel (Natriumhydroxid, Kaliumhydroxid) | q.s |
| Kreatinin | 0,1 |
| Kreatin | 0,5 |
| Natriumchlorid | 3,00 |
| Wasser | ad 100 |

| **Beispiel 6** | Gew.-% |
|---|---|
| Hydroxypropylguar | 0,05 |
| Acrylat/$C_{10-30}$ -Alkylacrylatcrosspolymer | 1,00 |
| Dinatriumphenyldibenzimidazoltetrasulfonat | 2,00 |
| Phenylbenzimidazolsulfonsäure | 2,00 |
| Isopropylpalmitat | 6,00 |
| Glycerin | 5,00 |
| Trinatrium EDTA | 1,00 |
| Konservierungsmittel | q.s |
| Parfüm, Farbstoffe | q.s |
| Neutralisationsmittel (Natriumhydroxid, Kaliumhydroxid) | q.s |
| Wasser | ad 100 |

**Vergleichsversuch**

Es wurden drei Rezepturen erstellt und auf Stabilität hinsichtlich Phasentrennung untersucht: Rezeptur B

| | |
|---|---|
| Isopropylpalmitat | 6,00 |
| Glycerin | 5,00 |
| Phenylbenzimidazolsulfonsäure | 2,00 |

(fortgesetzt)

| | |
|---|---|
| Dinatrumphenyldibenzimidazoltetrasulfonat | 2,00 |
| Acrylate /$C_{10-30}$ Alkylacrylat Crosspolymer | 1,00 |
| Konservierungsmittel | q.s |
| Parfüm, Farbstoffe | q.s |
| Neutralisationsmittel (Natriumhydroxid, Kaliumhydroxid) | q.s |
| Wasser | ad 100 |

Phasentrennung tritt auf.
Rezeptur C

| | |
|---|---|
| Isopropylpalmitat | 6,00 |
| Glycerin | 5,00 |
| Phenylbenzimidazolsulfonsäure | 2,00 |
| Dinatrumphenyldibenzimidazoltetrasulfonat | 2,00 |
| Hydroxypropyl guar | 0,05 |
| Konservierungsmittel | q.s |
| Parfüm, Farbstoffe | q.s |
| Neutralisationsmittel (Natriumhydroxid, Kaliumhydroxid) | q.s |
| Wasser | ad 100 |

Instabile Formulierung. Bei einer Einsatzkonzentration von 1,00 % Hydroxypyl guar ist auch die Formulierung instabil
Rezeptur D

| | |
|---|---|
| Isopropylpalmitat | 6,00 |
| Glycerin | 5,00 |
| Phenylbenzimidazolsulfonsäure | 2,00 |
| Dinatrumphenyldibenzimidazoltetrasulfonat | 2,00 |
| Hydroxypropyl guar | 0,05 |
| Acrylate /$C_{10-30}$ Alkylacrylat Crosspolymer | 1,00 |
| Konservierungsmittel | q.s |
| Parfüm, Farbstoffe | q.s |
| Neutralisationsmittel (Natriumhydroxid, Kaliumhydroxid) | q.s |
| Wasser | ad 100 |

Stabile Formulierung.

Nur die Rezeptur D, die die erfindungsgemäßen Merkmale trägt, ist stabil gegenüber Phasentrennung. Rezepturen B und C rahmen auf.

## Patentansprüche

1. Kosmetische Hydrodispersionen von Öltröpfchen in einer Wasserphase, welche höchstens 1 Gew.-%, bezogen auf das Gesamtgewicht der Zubereitungen, an Emulgatoren enthalten, bevorzugt aber weitgehend frei von Emulgatoren sind, wobei die wäßrige Phase aufweist:

(a) einen Gehalt an Hydroxypropylguar sowie
(b) einen Gehalt an einem oder mehreren Acrylatpolymeren, gewählt aus der Gruppe, die die INCI-Bezeichnung "Acrylates/$C_{10-30}$Alkyl Acrylate Crosspolymer" tragen.

2. Zubereitungen nach Anspruch 1, **dadurch gekennzeichnet, daß** die Konzentration an Hydroxypropylguar 0,001 bis 5 Gew.-% beträgt, besonders bevorzugt 0,05 - 2,5 Gew.-%, insbesondere 0,1 - 1 Gew.-%, bezogen auf das Gesamtgewicht der Zubereitung.

3. Zubereitungen nach Anspruch 1, **dadurch gekennzeichnet, daß** die Konzentration an einem oder mehreren Polyacrylaten, bevorzugt an Verbindungen, die die INCI-Bezeichnung "Acrylates/$C_{10-30}$-Alkyl Acrylate Crosspolymer" tragen, in den Zubereitungen gemäß der Erfindung 0,001 bis 10 Gew.-% beträgt, besonders bevorzugt 0,05 - 5 Gew.-%, insbesondere 0, 1 - 2,5 Gew.-%, bezogen auf das Gesamtgewicht der Zubereitung.

4. Zubereitungen nach Anspruch 1, **dadurch gekennzeichnet, daß** das Gewichtsverhältnis von Hydroxypropylguar zu einem oder mehreren Polyacrylaten, bevorzugt an Verbindungen, die die INCI-Bezeichnung "Acrylates/$C_{10-30}$-Alkyl Acrylate Crosspolymer" tragen, in den Zubereitungen gemäß der Erfindung aus dem Bereich von 100 : 1 bis 1 : 100 gewählt wird, bevorzugt 10 : 1 bis 1 : 50, insbesondere bevorzugt aus dem Bereich von 1 : 1 bis 1 : 20.

5. Zubereitungen nach Anspruch 1, **dadurch gekennzeichnet, daß** in ihrer wäßrigen Phase Elektrolyte enthalten sind, gewählt aus der Gruppe der Salze mit folgenden Anionen: Chloride, ferner anorganische Oxo-Element-Anionen, von diesen insbesondere Sulfate, Carbonate, Phosphate, Borate und Aluminate, ferner auf organischen Anionen basierende Elektrolyte, z.B. Lactate, Acetate, Benzoate, Propionate, Tartrate, Citrate, Aminosäuren Ethylendiamintetraessigsäure und deren Salze.

6. Zubereitungen nach Anspruch 1, **dadurch gekennzeichnet, daß** in ihrer wäßrigen Phase Elektrolyte enthalten sind, gewählt aus der Gruppe der Salze mit folgenden Kationen Ammonium,- Alkylammonium,- Alkalimetall-, Erdalkalimetall,- Magnesium-, Eisen- bzw. Zinkionen.

7. Zubereitungen nach Anspruch 1, **dadurch gekennzeichnet, daß** in ihrer wäßrigen Phase Elektrolyte enthalten sind, gewählt aus der Gruppe der wasserlöslichen, zumeist als Alkalisalze vorliegenden wasserlöslichen UV-Filtersubstanzen, insbesondere solche, die an ihrem Molekülgerüst eine oder mehrere Sulfonsäuregruppen bzw. Sulfonatgruppen tragen.

8. Zubereitungen nach Anspruch 5, 6 oder 7, **dadurch gekennzeichnet, daß** die Ionenstärke der wäßrigen Phasen, in der der oder die Elektrolyte gelöst vorliegen, mindestens 0,05 mol/l, insbesondere mindestens 0,075 mol/l, besonders bevorzugt mindestens 0,10 mol/l beträgt.

## Claims

1. Cosmetic hydrodispersions of oil droplets in a water phase which comprises at most 1% by weight, based on the total weight of the preparations, of emulsifiers, but are preferably largely free from emulsifiers, where the aqueous phase has:

(a) a content of hydroxypropylguar and
(b) a content of one or more acrylate polymers selected from the group which carries the INCI name "Acrylates/$C_{10-30}$-Alkyl Acrylate Crosspolymer".

2. Preparations according to Claim 1, **characterized in that** the concentration of hydroxypropylguar is 0.001 to 5% by weight, particularly preferably 0.05-2.5% by weight, in particular 0.1-1% by weight, based on the total weight of the preparation.

3. Preparations according to Claim 1, **characterized in that** the concentration of one or more polyacrylates, preferably of compounds which carry the INCI name "Acrylates/$C_{10-30}$Alkyl Acrylate Crosspolymer" in the preparations according to the invention is 0.001 to 10% by weight, particularly preferably 0.05-5% by weight, in particular 0.1-2.5% by weight, based on the total weight of the preparation.

4. Preparations according to Claim 1, **characterized in that** the weight ratio of hydroxypropylguar to one or more

polyacrylates, preferably to compounds which carry the INCI name "Acrylates/$C_{10-30}$Alkyl Acrylate Crosspolymer" in the preparations according to the invention is selected from the range from 100:1 to 1:100, preferably 10:1 to 1: 50, particularly preferably from the range from 1:1 to 1:20.

**5.** Preparations according to Claim 1, **characterized in that** in their aqueous phase electrolytes are present, selected from the group of the salts with the following anions: chlorides, also inorganic oxo element anions, of these in particular sulphates, carbonates, phosphates, borates and aluminates, also electrolytes based on organic anions, e.g. lactates, acetates, benzoates, propionates, tartrates, citrates, amino acids ethylenediaminetetraacetic acid and salts thereof.

**6.** Preparations according to Claim 1, **characterized in that** in their aqueous phase electrolytes are present, selected from the group of the salts with the following cations ammonium, alkylammonium, alkali metal, alkaline earth metal, magnesium, iron and zinc ions.

**7.** Preparations according to Claim 1, **characterized in that** in their aqueous phase electrolytes are present, selected from the group of the water-soluble UV filter substances, mostly water-soluble UV filter substances present as alkali metal salts, in particular those which carry one or more sulphonic acid groups or sulphonate groups on their molecular backbone.

**8.** Preparations according to Claim 5, 6 or 7, **characterized in that** the ionic strength of the aqueous phases in which the electrolyte or electrolytes are present in dissolved form is at least 0.05 mol/l, in particular at least 0.075 mol/l, particularly preferably at least 0.10 mol/l.

## Revendications

**1.** Hydrodispersions cosmétiques de gouttes d'huile dans une phase aqueuse, qui contiennent au plus 1% en poids, par rapport au poids total des préparations, d'émulsifiants, mais qui sont de préférence dans une large mesure exemptes d'émulsifiants, la phase aqueuse présentant :

(a) une teneur en hydroxypropylguar, ainsi que
(b) une teneur en un ou plusieurs polymères d'acrylate, choisis dans le groupe qui porte la dénomination INCI "Acrylates/$C_{10-30}$Alkyl Acrylate Crosspolymer".

**2.** Préparations selon la revendication 1, **caractérisées en ce que** la concentration en hydroxypropylguar est de 0,001 à 5% en poids, de manière particulièrement préférée de 0,05-2,5% en poids, en particulier de 0,1-1% en poids, par rapport au poids total de la préparation.

**3.** Préparations selon la revendication 1, **caractérisées en ce que** la concentration en un ou plusieurs polyacrylates, de préférence en composés qui portent la dénomination INCI "Acrylates/$C_{10-30}$-Alkyl Acrylate Crosspolymer", dans les préparations selon l'invention est de 0,001 à 10% en poids, de manière particulièrement préférée 0,05-5% en poids, en particulier 0,1-2,5 % en poids, par rapport au poids total de la préparation.

**4.** Préparations selon la revendication 1, **caractérisées en ce que** le rapport pondéral d'hydroxypropylguar à un ou plusieurs polyacrylates, de préférence aux composés qui portent la dénomination INCI "Acrylates/$C_{10-30}$-Alkyl Acrylate Crosspolymer", dans les préparations selon l'invention est choisi dans la plage de 100:1 à 1:100, de préférence de 10:1 à 1:50, en particulier de préférence dans la plage de 1:1 à 1:20.

**5.** Préparations selon la revendication 1, **caractérisées en ce qu'**elles contiennent dans leur phase aqueuse des électrolytes choisis dans le groupe des sels présentant les anions suivants : les chlorures, en outre les anions d'oxo-éléments inorganiques, parmi lesquels en particulier les sulfates, les carbonates, les phosphates, les borates et les aluminates, en outre des électrolytes à base d'anions organiques, par exemple les lactates, les acétates, les benzoates, les propionates, les tartrates, les citrates, les acides aminés, l'acide éthylènediaminetétraacétique et leurs sels.

**6.** Préparations selon la revendication 1, **caractérisées en ce que** leur phase aqueuse contient des électrolytes, choisis dans le groupe des sels présentant les cations suivants : ions d'ammonium, d'alkylammonium, de métal alcalin, de métal alcalino-terreux, de magnésium, de fer ou, selon le cas de zinc.

7. Préparations selon la revendication 1, **caractérisées en ce qu'**elles contiennent dans leur phase aqueuse des électrolytes, choisis dans le groupe des substances filtre des UV, solubles dans l'eau, se trouvant généralement sous forme de sels de métal alcalin, en particulier celles qui présentent un ou plusieurs groupes acide sulfonique ou sulfonate sur leur structure moléculaire.

8. Compositions selon la revendication 5, 6 ou 7, **caractérisées en ce que** la force ionique des phases aqueuses, dans lesquelles le ou les électrolytes se trouvent sous forme dissoute, vaut au moins 0,05 mole/l en particulier au moins 0,075 mole/l, de manière particulièrement préférée au moins 0,10 mole/l.